Europäisches Patentamt

European Patent Office

Office européen des brevets

(19)

(11) **EP 0 821 232 A1**

(12) **EUROPÄISCHE PATENTANMELDUNG**

(43) Veröffentlichungstag:
28.01.1998 Patentblatt 1998/05

(51) Int. Cl.$^6$: **G01N 33/487**, C12M 1/34,
G06F 15/80

(21) Anmeldenummer: 96890130.6

(22) Anmeldetag: 24.07.1996

(84) Benannte Vertragsstaaten:
**CH DE ES FR GB IT LI NL**

(71) Anmelder:
**SY-LAB Vertriebsges.m.b.H.
A-3002 Purkersdorf (AT)**

(72) Erfinder:
• **Pfützner, Helmut
5640 Badgastein (AT)**

• **Futschik, Karl
2344 Maria Enzersdorf (AT)**

(74) Vertreter:
**Müllner, Erwin, Dr. et al
Patentanwälte
Dr. Erwin Müllner
Dipl.-Ing. Werner Katschinka
Postfach 159
Weihburggasse 9
1010 Wien (AT)**

(54) **Verfahren zur Bestimmung von Bestandteilen einer Probe und Vorrichtung zur Durchführung des Verfahrens**

(57) Zur Bestimmung der Zellkonzentrationen, insbesondere des Wachstums von Mikroorganismenkulturen und allenfalls der Keimart von Mikroorganismen in bebrütbaren Meßgefäßen (2), werden als Kenngrößen Zeitwerte von kontinuierlich variablen Paramtern wie Leitwert, Impedanz, Trübungsgrad, pH-Wert und dergleichen, zu welchen bestimmte Ereignisse, wie etwa Schwellwertüberschreitungen, Kurvenmaxima, Minima, Wendepunkte und Extrema eintreten, als Schar oder Scharen von Eingangsgrößen einem Neuralen Netz (13) zugeführt. Das Neurale Netz (13) ist Teil eines Rechners und arbeitet mit einem keimspezifischen Vortraining und mit einem anwenderspezifischen Nachtraining unter Einsatz einer Referenzmethode. Es werden die Kenngrößen selbständig erfaßt und aus diesen eine Aussage über Art und Umfang der Mikroorganismen und die Bedeutung der Kenngrößen für die Bewertung im Rechner abgeleitet. Die Vorrichtung zur Durchführung des Verfahrens umfaßt Meßgefäße (2) in einem Thermoblock (1). Über Elektroden (3, 4) werden zyklisch Meßwerte erfaßt und in einem Kennwertdetektor (9, 10, 11) werden die Zeitwerte (t) von Ereignissen wie Kurvenmaxima, Minima, Wendepunkte, Anstiege oder Schwellenwerte ermittelt. Vom Kennwertdetektor (9, 10, 11) empfängt das nachgeschaltete Neurale Netz (13) diese Zeitwerte (t) bzw. allenfalls über Inverter (15) invertierte Werte (1/t) oder auch über Differenzbildner (16) gebildete Differenzen derselben zur Auswertung im Rechner.

*Fig.1*

EP 0 821 232 A1

## Beschreibung

Die Erfindung betrifft ein Verfahren zur Bestimmung von Bestandteilen einer Probe mit meßtechnischer Erfassung des Zeitverlaufes mindestens eines physikalischen Parameters, beispielsweise der Trübung oder der Impedanz, wobei aus den ermittelten Werten Kenngrößen abgeleitet und einem Neuralen Netz zugeführt werden. Zur Bestimmung von Blut- oder Harnwerten ist es aus der EP-A1-581 023 bekannt, von Meßwerten R ausgehend, ein analytisches Resultat A zu bestimmen. Die Meßwerte bzw. auch die daraus abgeleiteten Kennwerte ("Meßresultate") werden an den Eingang eines Neuralen Netzes gelegt. Der Ausgangswert A entspricht z.B. der Konzentration eines bestimmten gelösten Stoffes. Dabei werden auch zeitliche Veränderungen der Meßwerte R berücksichtigt.

Zur Bestimmung der Zellkonzentration von Mikroorganismen in Proben, wie sie beispielsweise von Lebensmittelprüfanstalten gezogen oder im Bereich der Nahrungsmittelindustrie sowie etwa auch in der Milchwirtschaft von den Unternehmen selbst durchgeführt wird, bedient man sich weitgehend automatisierter verfahren und Vorrichtungen, um die hohe Anzahl von Proben in rationeller Weise meßtechnisch zu erfassen. So werden Proben in Thermoblöcken oder Klimakammern bei Temperaturen, die auf die Messungen abgestimmt sind, aufgestellt und Parameter wie der Trübungsgrad, der pH-Wert, die elektrische Leitfähigkeit und bzw. oder die Impedanz optisch bzw. durch Sensoren, wie beispielsweise Elektroden, in der bebrüteten Kulturflüssigkeit zyklisch gemessen. Die Messungen werden über einen Rechner gesteuert und automatisch durchgeführt. Ausgewertet werden zeitliche Veränderungen dieser Parameter durch Kurven über der Zeitachse. Charakteristisch ist vielfach jene Zeit (Detektionszeit $t_s$), zu der die durch das Keimwachstum verursachte Veränderung eines der Parameter einen vorgegebenen Schwellwert erreicht. Die Funktion $C(t_s)$ der Zellkonzentration C in Abhängigkeit von der Detektionszeit ($t_s$) hängt von der Keimart ab, jedoch spielen auch eine Reihe von Kulturparametern eine Rolle. Die Konzentrationen der Stoffe, welche die Nährlösung enthält, die Temperatur der Bebrütung und andere biologische und technologische Parameter können die Zellkonzentration maßgebend beeinflussen. Der jeweilige Anwender ist damit gezwungen, die Funktion betriebsspezifisch selbst zu bestimmen.

Die Effizienz der resultierenden Kalibrierfunktion $C(t_s)$ wird ihrerseits durch kausal kaum beschreibbare Artefakte beeinflußt. Aufgrund systematischer Einflüsse können letztere weitgehend reproduzierbar ausfallen. Durch eine einfache Korrektur von $C(t_s)$ können sie aber kaum unwirksam gemacht werden.

Aufgrund unterschiedlicher physikalischer Mechanismen erbringen die einzelnen Parameter oftmals stark differierende Detektionszeiten ($t_s$). Vorteilhafterweise wählt man zur Detektion eines bestimmten Keimes jenen Parameter, der erfahrungsgemäß kürzestes $t_s$ erbringt. Bei der Auswahl ergeben sich dabei Kompromisse wegen konzentrationsabhängiger Optima, wegen unterschiedlicher meßtechnischer Vor- und Nachteile usw. Vor allem aber bleibt bei Übergang von einem zu einem anderen Parameter der Informationsgehalt des ersten ungenutzt. Die Gesamtheit der Informationen auszunützen scheitert meist daran, daß komplexe Zusammenhänge sich einer definierten Beschreibbarkeit entziehen.

Die schon erwähnte Abhängigkeit eines Parameters von der Zeit bietet prinzipiell die Möglichkeit, aus dem Funktionsverlauf auf die Keimart rückzuschließen. Die Erkennung relevanter Charakteristika erfordert rein visuell viel Erfahrung und ist auf automatischer Weise sehr schwierig, da komplexe Probleme der Mustererkennung vorliegen.

Eine Möglichkeit, vermehrte Information zu erarbeiten, ergibt sich aus Mehrfachansätzen in unterschiedlichen Nährmedien bzw. bei unterschiedlichen chemophysikalischen Bedingungen (z.B. unterschiedlicher Pufferung). Das Problem der effektiven Auswertung fällt dabei aber noch größer aus.

Die Erstellung von Algorithmen zur Ableitung von logischen Schlüssen aus dem Informations-Überangebot entartet - insbesondere wegen des Mitwirkens betriebsspezifischer Paramter und Artefakte - zu einem im Rechner kaum lösbaren Problem. Daher begnügt man sich mit der selbsttätigen Aufzeichnung der Parameter über der Zeitachse bei entsprechender Zyklus- und Temperatursteuerung des Systems. Die Auswertung obliegt der Erfahrung des Menschen. Im Falle einer vorgegebenen Keimart wird sie häufig dadurch unterstützt, daß die betriebsspezifische selbst bestimmte Kalibrierfunktion im Rechner implementiert wird. Anstelle der Detektionszeit wird dabei vielfach auch vom Auftreten eines vorgebbaren zeitlichen Anstieges des ausgewerteten Parameters ausgegangen. Die dermaßen automatisierte Auswertung basiert damit aber auf der Nutzung einer einzigen Kenngröße und läßt den von der Gesamtheit des Zeitverlaufes - bzw. der Zeitverläufe aller meßtechnisch erfaßten Parameter - repräsentierten hohen Informationsgehalt ungenutzt.

Die Erfindung zielt darauf ab, den gesamten hier vorliegenden Informationsgehalt ohne Inanspruchnahme der menschlichen Erfahrung auf automatische Weise zu nutzen und die Zuverlässigkeit der Zellkonzentrationsbestimmung damit entscheidend zu steigern. Dies wird dadurch erreicht, daß zur Bestimmung von Zellkonzentrationen, insbesondere des Wachstums von Mikroorganismenkulturen in bebrüteten Meßgefäßen, als Kenngrößen des oder der zeitabhängigen Parameter Zeitwerte, zu denen bestimmte Ereignisse, wie Schwellwertüberschreitungen, Wendepunkte oder Extrema, auftreten, als Schar bzw. Scharen von Eingangsgrößen dem Neuralen Netz zugeführt werden. Es werden also vorzugsweise nicht die zu unterschiedli-

chen Meßzeitpunkten $t_1$ ..... $t_n$ auftretenden Meßwerte P genutzt, sondern die bestimmten Ereignissen (z.B. Überschreitungen von Schwellwerten $P_1$ ..... $P_n$) entsprechenden Zeitwerte zu denen bestimmte Kurvencharakteristika (z.B. Wendepunkte, Maxima etc.) auftreten. An das Neurale Netz werden somit erfindungsgemäß spezifische Zeitwerte $t_x$ (und darüber hinaus auch Codezahlen $C_x$) gelegt. Es ist zweckmäßig, wenn bei Mehrfachansätzen zur Bestimmung sowohl der Zellkonzentrationen als auch der Art der Mikroorganismen dem Neuralen Netz entsprechend viele Scharen von Zeitwerten gleichzeitig zugeführt werden, bei einem Doppelansatz und zwei Parametern also insgesamt vier Scharen. Ferner sieht die Erfindung vor, daß dem Netz zur Berücksichtigung von während der Meßzeit nicht erreichten Schwellwerten oder Wendepunkten statt den Zeitwerten vorzugsweise die zu ihnen inversen Reaktionsschnellen $r = 1/t$ zugeführt werden. Es kann nämlich das Problem auftreten, daß einzelne Kennwerte während der vorgesehenen Meßzeit nicht detektiert werden. Eine Ursache dafür kann sein, daß die betreffenden Organismen nur sehr langsam anwachsen, eine andere, daß der betrachtete Parameter sich trotz starkem Anwachsen der Organismen sich so schwach verändert, daß ein hoch angesetzter Schwellwert prinzipiell nie erreicht wird. Der entsprechende, dem Netz zugeführte Zeitwert t entartet damit zu Unendlich. Insbesondere für derartige Anwendungsbereiche ist demnach vorgesehen, dem Netz statt der Zeitwerte t die dazu reziproken Reaktionsschnellen $r = 1/t$ zuzuführen. Als Vorteil ergibt sich, daß der Wertebereich von r prinzipiell endlich groß ist. Es können zur Klassifizierung auch der Art der Mikroorganismen dem Netz auch die Differenzen der Reaktionsschnellen von Kennwerten unterschiedlicher Parameter zugeführt werden.

Die erwähnten Individualwerte als Kenngrößen ermöglichen es, daß dem Neuralen Netz weitere für mehrere Proben gemeinsam gültige Betriebsbedingungen in Form von physikalischen Größen wie Temperatur, Begasungsdruck, und von Codezahlen für Nährlösungsart, Meßgefäßtype, Elektrodentype, Laborant und dergleichen zugeführt werden. Diese weiteren Betriebsbedingungen können vom Zeitverlauf unabhängig sein. Auch derartige Faktoren können einen Einfluß auf die Analyse haben. Erst die Tatsache, daß das Netz nicht Kurvenverläufe sondern Kenngrößen verarbeitet, gestattet die Einzbeziehung der vorgenannten Randbedingungen. Die tatsächliche Relevanz der letzteren läßt sich mittels des trainierten Neuralen Netzes überprüfen, indem der Wert des zur Diskussion stehenden Faktors variiert wird. Bleibt dies ohne Auswirkung auf die Ausgangsgrößen, so kann der Faktor bzw. die Größe aus der Berücksichtigung ausgeschieden werden. Was das Neurale Netz betrifft, so können die aus einem keimspezifischen Vortraining resultierenden Netzgewichte durch ein anwenderspezifisches Nachtraining anwenderspezifisch modifiziert werden. Die vorgenannte Aussage wird insbesondere auf der Basis des Vor- und Nachtrainings, vorzugsweise aus selbsttätig selektierten Vergleichsversuchen aus dem Vortrainings- und dem durch sämtliche Kenngrößen ergänzten Nachtrainingsbereich erhalten.

Die Vorrichtung zur Durchführung des Verfahrens bei der Meßgefäße vorgesehen sind, denen Strahlungsquellen und Sensoren, z.B. für Trübungsmessungen, und Elektroden bzw. Meßwertgeber im Inneren, z.B. für Impedanz- oder pH-Wertmessungen, zugeordnet sind, wobei die Sensoren, Elektroden bzw. Meßwertgeber an Kennwertdetektoren für die Ermittlung von Kenngrö-ßen, z.B. von Schwellwerten, Extremwerten oder Wendepunkten, angeschlossen sind und diese Kennwertdetektoren einer Auswerteschaltung angehören, welche ein Neurales Netz umfaßt, ist dadurch gekennzeichnet, daß die Meßgefäße für Nährlösungen und Biosubstanzen in einer temperaturregelbaren Zone, z.B. einem Thermoblock oder einer Klimakammer, vorgesehen sind, daß als Kennwertdetektoren Schwellwertdetektoren zur Erfassung von variablen Zeitpunkten ($t_{s1}$, $t_{s2}$) bei Erreichen eines vorbestimmten Meßwertes und bzw. oder Wendepunktdetektoren zur ausschließlichen Erfassung von Zeitpunkten ($t_w$) des Auftretens von Wendepunkten und bzw. oder Extremwertdetektoren zur ausschließlichen Erfassung von Zeitpunkten des Auftretens von Extremwerten vorgesehen sind und daß das Neurale Netz für selbsttätige Zuordnungen zur Bestimmung von Umfang und Art der Mikroorganismen als Funktion der Kenngrößen trainiert ist. Dabei werden alle von den Kennwertdetektoren kommenden Werte für selbsttätige Zuordnungen genutzt. Abgesehen von den Eingängen für die Kenngrößen ist es vorteilhaft, wenn die Auswerteschaltung einen Eingang für insbesondere über eine Tastatur eingegebene zeit- oder schwellwertunabhängige Daten und Codes umfaßt. Wie schon erwähnt, sind darunter Daten über Art, Herkunft und Zusammensetzung einer Nährlösung, Umgebungsdaten oder dergleichen Einzelinformationen zu verstehen. Insbesondere dann, wenn einzelne Meßwerte während der vorgesehenen Meßzeiten nicht erreicht werden, dann können den Schwellwertdetektoren oder Wendepunktdetektoren Inverter zur Erfassung der zu den Zeitwerten ($t_{s1}$, $t_{s2}$, $t_w$) inversen Reaktionsschnellen ($r_{s1}$, $r_{s2}$, $r_w$) nachgeschaltet sein. Für eine Klassifizierung ist es zweckmäßig, wenn den Invertern Differenzbildner zur Erfassung der Differenzen ($r_{s1,2} - r_{s1,1}$, $r_{s2,2} - r_{s2,1}$, $r_{w,2} - r_{w,1}$, $r_{w,1} - r_{s1,1}$, $r_{s2,1} - r_{s1,1}$, $r_{w,2} - r_{s1,2}$, $r_{s2,2} - r_{s1,2}$) nachgeschaltet sind.

Neurale Netze umfassen lernfähige Neuronengruppierungen, die zugeführte Informationen nicht nur systemgerecht zu einem Ergebnis verarbeiten, sondern die Informationen und Ergebnisse in ihr eigenes System einbringen, um diese in Zukunft bei einer Datenverarbeitung mitzuberücksichtigen bzw. das System auch im Programmablauf zu aktualisieren. Es ist hier zweckmäßig, wenn das Neurale Netz im wesentlichen mit sigmoiden Übertragungsfunktionen, jedoch mit linearen Übertragungsfunktionen in der Ausgangsschicht ausgeführt ist. Im Sinne geringer Verarbeitungszeiten bzw. auch kompakter Ausführung des Meßsystems ist es zweckmäßig, das Neurale Netz hardwaremäßig aufzubauen, es kann aber auch unter Einsatz eines herkömmlichen Rechners implementiert werden.

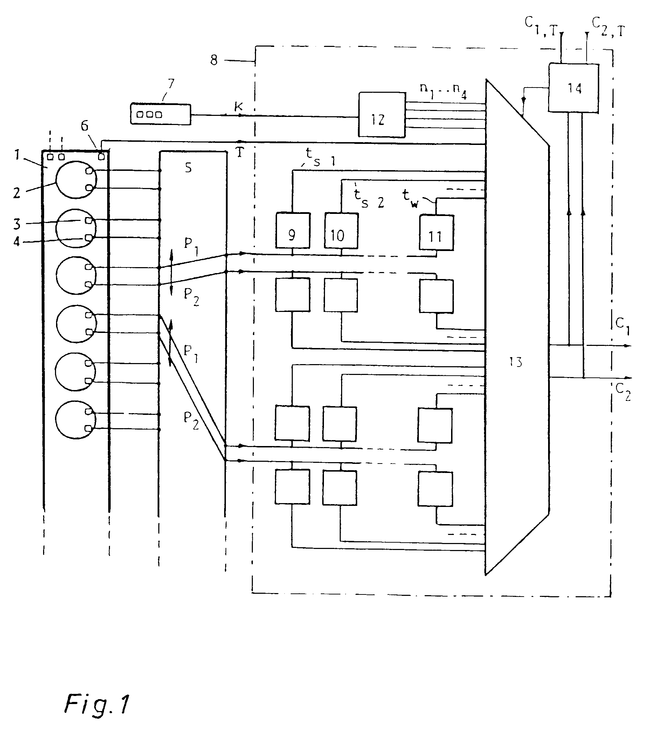

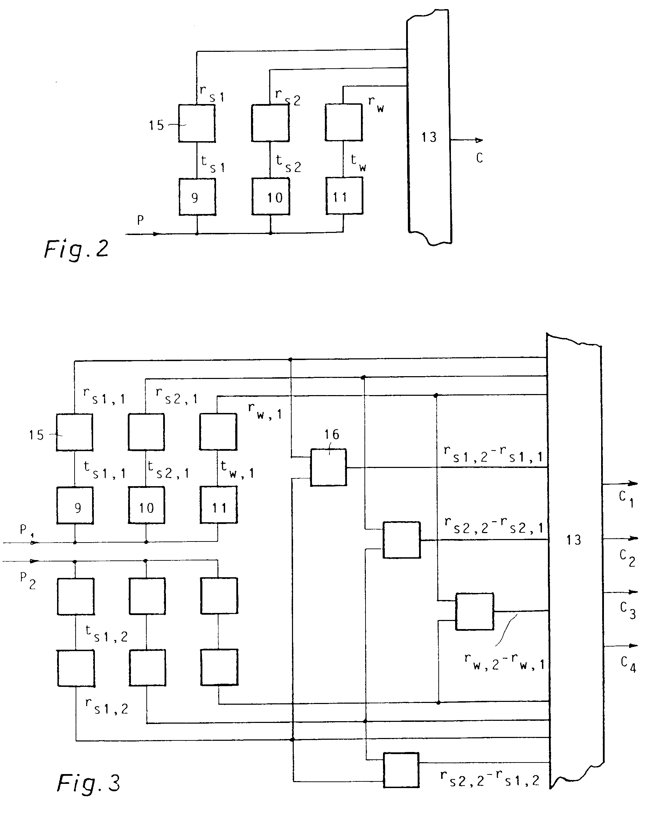

Ausführungsbeispiele zu der erfindungsgemäßen Vorrichtung sind in den Zeichnungen in Form von Prinzipschaltungen dargestellt. Fig. 1 zeigt ein Prinzipschaltbild einer Vorrichtung zur Bestimmung der Zellkonzentration, Fig. 2 eine erste und Fig. 3 eine zweite Möglichkeit der Erweiterung der Anordnung nach Fig. 1.

In einem beheizbaren Thermoblock 1 z.B. aus Aluminium mit parallelen, senkrechten Bohrungen sind Meßgefäße 2 eingeschoben, die mikrobiologische Proben in einer Nährlösung enthalten. Es soll z.B. festgestellt werden, ob schädliche Keime in einer Milchprobe einer Herkunftsquelle enthalten sind und um welche Keime es sich handelt. In den Meßgefäßen sind Elektroden 3, 4 z.B. aus Stahl oder ähnliche Sensoren zur Messung der Zeitverläufe, etwa der elektrischen Leitfähigkeit $P_1$ und des pH-Werts $P_2$. Durch einen Multiplexer 5 werden diese hier beispielsweise genannten zwei Parameter $P_1$ und $P_2$ in zyklischer Weise für jeweils zwei benachbarte Meßgefäße 2 erfaßt, die im Sinne eines Doppelansatzes gleiche Keimtypen, jedoch unterschiedliche Nährlösungen beinhalten. Über einen Thermistor 6 erfolgt die Erfassung der allen Zellen gemeinsamen Temperatur. Eine Tastatur 7 ermöglicht die Eingabe von Daten über die Nährlösungskomponenten etwa in Form einer Codezahl K.

Die Elektroden 3, 4 sind über den genannten Multiplexer 5 mit einer Auswerteschaltung 8 verbunden, die Kennwertdetektoren 9, 10, 11 enthält. Diese selektieren Schwellwerte in Form von Zeitinformationen $t_{s1}$, $t_{s2}$, also von Zeitpunkten, da vorbestimmte Schwellwerte etwa der Leitfähigkeit erreicht werden. Es handelt sich dabei um Schwellwertdetektoren 9, 10 bzw. auch um Wendepunktdetektoren 11, letztere zur Erfassung der Zeiwerte $t_w$ des Auftretens von Wendepunkten bei den Meßwerten. Alle Detektoren 9, 10, 11 sind vierfach ausgeführt, in Entsprechung zu zwei Parametern $P_1$, $P_2$ für jeweils zwei Zellen 2.

Der Tastatur 7 ist ein Datenspeicher 12 nachgeschaltet, der unter anderem einer eingegebenen Codezahl K vier, die zwei Nährlösungstypen charakterisierende Konzentrationsgrößen ($n_1$ bis $n_4$) zuordnet.

Sämtliche aus $P_1$, $P_2$, T und K abgeleitete Größen werden innerhalb der Auswerteschaltung 8 einem Neuralen Netz 13 als Eingangsgrößen zugeführt, womit sich für die Eingangsschicht siebzehn Neuronen ergeben. Die zwei Neuronen aufweisende Ausgangsschicht liefert als Ausgangsgrößen die Anfangskonzentrationen $C_1$ und $C_2$ von zwei möglichen Keimtypen.

Die Auswerteschaltung 8 inkludiert ein Informationsladegerät 14, an dessen Eingängen neben der Rückkoppelung der eben ermittelten Anfangskonzentrationen $C_1$ und $C_2$ ferner noch die Trainingsgrößen $C_{1,T}$ und $C_{2,T}$ liegen. Letztere können mittels der bekannten Plattengußmethode als Referenzmethode ermittelt werden. Das Informationsladegerät 14 verarbeitet seine Eingangsgrößen über einen Trainingsalgorithmus und ist an das Neurale Netz 13 angeschlossen.

Grundsätzlich ist davon auszugehen, daß die Eingangsgrößen des Neuralen Netzes in erster Linie aus der Funktion P(t) abgeleitet werden. Dabei können beispielsweise die für äquidistante Zeitwerte t anfallenden Werte P als Eingangsgrößen verwendet werden. Da die Detektionszeit um Größenordnungen differieren kann, wird vorzugsweise aber die Umkehrfunktion t(P) genutzt, indem z.B. für fünf vorgegebene Schwellwerte die entsprechenden fünf Werte $t_s$ als Eingangsgrößen verwendet werden. Zwecks Ausnützung möglichst umfangreichen Informationsgehaltes ist vorgesehen, Scharen von Eingangsgrößen aller meßtechnisch erfaßten Parameter gleichzeitig zu verarbeiten (z.B. 15 Größen für drei Parameter $P_1$, $P_2$, $P_3$).

Auch bei der Nutzung der Umkehrfunktion t(P) ergibt sich erfahrungsgemäß das Problem, daß unterschiedlichen Abschnitten von t(P) verschieden starker Informationsgehalt zukommt. Zwecks optimaler Nutzung der vorliegenden Information bei vertretbar hohem Aufwand bezüglich der Anzahl der Eingangsgrößen ist erfindungsgemäß vorgesehen, t nicht nur für starr vorgegebene Werte P abzuleiten, sondern auch spezifische, durch einfache Schaltungen bzw. Algorithmen leicht erfaßbare Kennwerte zu nutzen. So werden, wie bereits ausgeführt, jene Zeitwerte ermittelt, bei denen P ein Maximum, ein Minimum bzw. einen Wendepunkt aufweist. Bei drei erfaßten Parametern ergeben sich entsprechend am Eingang des Neuralen Netzes neun weitere Größen, um ein Maximum an Information zu berücksichtigen.

Wegen des oben erwähnten Problems der oft nicht durchschaubaren Mitwirkung verschiedenster Einflußparameter werden weitere Eingangsgrößen angesetzt, die für mehrere Proben gemeinsam gültige Betriebsbedingungen betreffen, also z.B. die Temperatur oder den Druck einer allfälligen Probenbegasung. Während diese Parameter durch Sensoren automatisch erfaßt werden können, wird es sinnvoll sein, die weiteren Informationen händisch als Eingangsgrößen bzw. Codezahlen vorzugeben. Derartige Codezahlen können, wie beim Ausführungsbeispiel, die Konzentration verschiedener Nährstoffkomponenten oder Ausgangsbedingungen (und damit die Ausgangsaktivität des Keims) kennzeichnen. Sie können aber auch scheinbar bedeutungslose Umstände, wie etwa den jeweiligen Nährlösungs-Hersteller (zur Berücksichtigung geringer Unterschiede der Komposition) berücksichtigen. Sich als irrelevant ergebende Eingangsgrößen können später als konstant angesetzt werden.

Als Netzausgangsgröße wird vorzugsweise die vor allem interessierende, zum Bebrütungsbeginn (Zeitpunkt t = 0) vorliegende Zellkonzentration C angesetzt. Erfindungsgemäß ist aber auch vorgesehen, zusätzliche Informationen anzusetzen, insbesondere dann, wenn mehrere Parameter P zur Verfügung stehen. Beispielsweise werden bei einem Nährmedium, welches das Wachstum von zwei Keimtypen zuläßt, Größen $C_1$ und $C_2$ den jeweiligen Teilkonzentrationen der beiden Keimtypen zugeordnet. Ebenso können Ausgangsgrößen auch Informationen repräsentieren, die sich aus Abweichungen vom zu erwartenden Funktionsverlauf ergeben und damit auf spezielle Keimeigenschaften hinweisen. Entsprechend können diese Abweichungen auch zur Bestimmung verschiedener, in der Nährlösung vorhandener Stoffe, wie z.B. Hemmstoffe, Wuchsstoffe oder Antibiotika, verwendet werden. Für den Fall von Mehrfachansätzen ist vorgesehen, ein Netz zu trainieren, dem sämtliche Informationen aller parallel angesetzten Proben gleichzeitig zugeführt werden. Daß heißt, daß beispielsweise bei einem Dreifachansatz unter Erfassung von zwei Parametern sechs Scharen von Eingangsgrößen verarbeitet werden.

Das Training des Netzes erfolgt grundsätzlich auf der Grundlage von unter standardisierten Bedingungen erarbeitetem keimspezifischen Erfahrungsmaterial. Die Gewichte (bzw. Biases) vortrainierter Netze werden unter anwenderspezifischen Bedingungen im Sinne eines Nachtrainings - unter Einsatz von Kontrollverfahren (z.B. Plattengußverfahren) zur Bestimmung von C - den speziellen Bedingungen angepaßt.

Fig. 2 betrifft eine Möglichkeit der Erweiterung des Ausführungsbeispiels nach Fig. 1, die dann sinnvoll ist, wenn einzelne Kennwerte während der vorgesehenen Meßzeit etwa wegen des langsamen Anwachsenes nicht detektierbar sind bzw. wenn ein Schwellwert zu hoch angesetzt ist. Dem Neuralen Netz werden dann nicht die zu unendlich entarteten Zeitwerte t, sondern die dazu reziproken Reaktionsschnellen r = 1/t zugeführt, die immer endlich groß sind. Zwischen den Kennwertdetektoren 9, 10, 11 und dem Netz 13 werden hier Inverter 15 eingeschaltet, welche die Reaktionsschnellen $r_{s1}$, $r_{s2}$, $r_w$ liefern.

Bei Auswertung der Reaktionsschnellen r ergibt sich letztlich auch eine einfache Möglichkeit zur Klassifizierung mehrerer Keimarten, auch ohne Mehrfachansatz. Unterschiedliche Arten - bzw. die interessierende Art und nicht interessierende Begleitkeime - sind meist dadurch unterscheidbar, daß die Schwellwerte oder Wendepunkte der einzelnen Parameter P in spezifischer Weise zu versetzten Zeitpunkten t und damit auch mit unterschiedlichen Reaktionsschnellen r ausfallen. Erfindungsgemäß ist vorgesehen, dem Netz die Differenzen der Reaktionsschnellen zuzuführen, die sich zwischen den einzelnen Parametern ergeben. Für drei erfaßte Parameter $P_1$, $P_2$ und $P_3$ werden dem Netz also beispielsweise für drei Kennwerte die sechs auf $P_1$ bezogenen Differenzen $r_{s1,2} - r_{s1,1}$, $r_{s1,3} - r_{s1,1}$, $r_{s2,2} - r_{s2,1}$, $r_{s2,3} - r_{s2,1}$, $r_{w,2} - r_{w,1}$, $r_{w,3} - r_{w,1}$ zugeführt. Zusätzlich können dem Netz erfindungsgemäß auch die innerhalb eines Parameters auftretenden Differenzen $r_{w,1} - r_{s1,1}$, $r_{s2,1} - r_{s1,1}$, $r_{w,2} - r_{s1,2}$, $r_{s2,2} - r_{s1,2}$, $r_{w,3} - r_{s1,3}$, $r_{s2,3} - r_{s1,3}$ zugeführt werden. Letzteres ermöglicht grobe Klassifizierungen selbst dann, wenn nur ein einziger Parameter erfaßt wird.

Als Netzausgang kann - wie oben beschrieben - jeder Keimart eine Anfangskonzentration C zugeordnet werden. Bei einer Klassifizierung bezüglich vier verschiedener Keimarten entsprechend $C_1$ bis $C_4$ wird bei Vorliegen der Keimart 2 damit $C_2$ endlich ausfallen. $C_1$, $C_3$ und $C_4$ hingegen werden gegen Null tendieren. Interessiert - wie im Falle von Salmonellen - nur eine Ja/Nein-Entscheidung im Sinne von "Vorliegen von Salmonellen" bzw. "kein Vorliegen", so kann dem ersten Fall beim Training $C_1 = 100$, $C_2 = 0$ und dem zweiten Fall $C_1 = 0$, $C_2 = 100$ zugeordnet werden. Beim Betrieb des Netzes kommt der dann kontinuierlich genutzten Skala 0 .... 100 die Bedeutung der Verläßlichkeit (Wahrscheinlichkeit) einer getroffenen Klassifizierung zu.

Bild 3 zeigt eine Möglichkeit der entsprechenden Erweiterung des Ausführungsbeispiels von Bild 2 bzw. Bild 1. Zwischen den Invertern 15 und dem Netz 13 werden hier Differenzbildner 16 eingeschaltet, welche für den hier skizzierten Fall von zwei Parametern $P_1$ und $P_2$ die Differenzen $r_{s1,2} - r_{s1,1}$, $r_{s2,2} - r_{s2,1}$, $r_{w,2} - r_{w,1}$ der Reaktionsschnellen $r_{s1}$, $r_{s2}$, $r_w$ liefern. Die Möglichkeit, auch innerhalb eines Parameters - z.B. $P_2$ - Differenzen anzusetzen, ist am Beispiel der Differenz $r_{s2,2} - r_{s1,2}$ dargestellt.

**Patentansprüche**

1. Verfahren zur Bestimmung von Bestandteilen einer Probe mit meßtechnischer Erfassung des Zeitverlaufes mindestens eines physikalischen Parameters, beispielsweise der Trübung oder der Impedanz, wobei aus den ermittelten Werten Kenngrößen abgeleitet und einem Neuralen Netz zugeführt werden, **dadurch gekennzeichnet,** daß zur Bestimmung von Zellkonzentrationen, insbesondere des Wachstums von Mikroorganismenkulturen in bebrüteten Meßgefäßen, als Kenngrößen des oder der zeitabhängigen Parameter Zeitwerte, zu denen bestimmte Ereignisse, wie Schwellwertüberschreitungen, Wendepunkte oder Extrema, auftreten, als Schar bzw. Scharen von Eingangsgrößen dem Neuralen Netz zugeführt werden.

2. Verfahren nach Anspruch 1, **dadurch gekennzeichnet,** daß bei Mehrfachansätzen zur Bestimmung sowohl der Zellkonzentrationen als auch der Art der Mikroorganismen dem Neuralen Netz entsprechend viele Scharen von Zeitwerten gleichzeitig zugeführt werden, bei einem Doppelansatz und zwei Parametern also insgesamt vier Scharen.

3. Verfahren nach einem der Ansprüche 1 und 2, **dadurch gekennzeichnet,** daß dem Netz zur Berücksichtigung von während der Meßzeit nicht erreichten Schwellwerten oder Wendepunkten statt den Zeitwerten vorzugsweise die zu ihnen inversen Reaktionsschnellen zugeführt werden.

4. Verfahren nach einem der Ansprüche 1 bis 3, **dadurch gekennzeichnet,** daß zur Klassifizierung auch der Art der Mikroorganismen dem Netz auch die Differenzen der Reaktionsschnellen von Kennwerten unterschiedlicher Parameter zugeführt werden.

5. Verfahren nach Anspruch 1, **dadurch gekennzeichnet,** daß dem Neuralen Netz weitere für mehrere Proben gemeinsam gültige Betriebsbedingungen in Form von physikalischen Größen, wie Temperatur oder Begasungsdruck, und in Form von Codezahlen für Nährlösungsart, Meßgefäßtype, Elektrodentype, Laborant u. dgl. zugeführt werden.

6. Verfahren nach einem der Ansprüche 1 bis 3, **dadurch gekennzeichnet,** daß ein keimspezifisches Vortraining vorgenommen wird und unter Einsatz einer Referenzmethode ein anwenderspezifisches Nachtraining erfolgt.

7. Vorrichtung zur Durchführung des Verfahrens nach einem der Ansprüche 1 bis 6, bei der Meßgefäße vorgesehen sind, denen Strahlungsquellen und Sensoren, z.B. für Trübungsmessungen, und Elektroden bzw. Meßwertgeber im Inneren, z.B. für Impedanz- oder pH-Wertmessungen, zugeordnet sind, wobei die Sensoren, Elektroden bzw. Meßwertgeber an Kennwertdetektoren für die Ermittlung von Kenngrößen, z.B. von Schwellwerten, Extremwerten oder Wendepunkten, angeschlossen sind und diese Kennwertdetektoren einer Auswerteschaltung angehören, welche ein Neurales Netz umfaßt, **dadurch gekennzeichnet,** daß die Meßgefäße (2) für Nährlösungen und Biosubstanzen in einer temperaturregelbaren Zone, z.B. einem Thermoblock (1) oder einer Klimakammer, vorgesehen sind, daß als Kennwertdetektoren (9, 10, 11) Schwellwertdetektoren (9, 10) zur Erfassung von variablen Zeitpunkten ($t_{s1}$, $t_{s2}$) bei Erreichen eines vorbestimmten Meßwertes und bzw. oder Wendepunktdetektoren (11) zur Erfassung von Zeitpunkten ($t_w$) des Auftretens von Wendepunkten und bzw. oder Extremwertdetektoren zur Erfassung von Zeitpunkten des Auftretens von Extremwerten vorgesehen sind und daß das Neurale Netz (13) für selbsttätige Zuordnungen zur Bestimmung von Umfang und Art der Mikroorganismen als Funktion der Kenngrößen trainiert ist.

8. Vorrichtung nach Anspruch 7, **dadurch gekennzeichnet,** daß die Auswerteschaltung (8) einen Eingang für insbesondere über eine Tastatur (7) eingegebene zeit- oder schwellwertungabhänige Daten und Codes umfaßt.

9. Vorrichtung nach Anspruch 7, **dadurch gekennzeichnet,** daß den Schwellwertdetektoren (9, 10) oder Wendepunktdetektoren (11) Inverter (15) zur Erfassung der zu den Zeitwerten ($t_{s1}$, $t_{s2}$, $t_w$) inversen Raktionsschnellen ($r_{s1}$, $r_{s2}$, $r_w$) nachgeschaltet sind.

10. Vorrichtung nach Anspruch 9, **dadurch gekennzeichnet,** daß den Invertern (15) Differenzbildner (16) zur Erfassung von Reaktionsschnellen-Differenzen ($r_{s1,2}$ - $r_{s1,1}$, $r_{s2,2}$ -$r_{s2,1}$, $r_{w,2}$ - $r_{w,1}$, $r_{w,1}$ - $r_{s1,1}$, $r_{s2,1}$ - $r_{s1,1}$, $r_{w,2}$ -$r_{s1,2}$, $r_{s2,2}$ - $r_{s1,2}$) nachgeschaltet sind.

Fig.1

Fig.2

Fig.3

8

Europäisches
Patentamt

**EUROPÄISCHER RECHERCHENBERICHT**

Nummer der Anmeldung

EP 96 89 0130

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,X | DE-A-42 24 621 (BOEHRINGER MANNHEIM GMBH) 27.Januar 1994 * das ganze Dokument * --- | 1-4,6,7, 9,10 | G01N33/487 C12M1/34 G06F15/80 |
| A | EP-A-0 647 707 (SY LAB VERTRIEBSGES M B H) 12.April 1995 * Seite 2, Spalte 1, Zeile 1 - Zeile 31 * --- | 7 | |
| A | US-A-5 338 659 (KAUVAR LAWRENCE M  ET AL) 16.August 1994 * Zusammenfassung * * Spalte 6, Zeile 47 - Zeile 56 * --- | 1,7 | |
| A | US-A-5 252 829 (NYGAARD LARS  ET AL) 12.Oktober 1993 * Zusammenfassung * ----- | 1,7 | |

RECHERCHIERTE
SACHGEBIETE (Int.Cl.6)

G01N
C12M
G06F

Der vorliegende Recherchenbericht wurde für alle Patentansprüche erstellt

| Recherchenort | Abschlußdatum der Recherche | Prüfer |
|---|---|---|
| DEN HAAG | 22.November 1996 | Scheu, M |

EPO FORM 1503 03.82 (P04C03)